Europäisches Patentamt

(19) European Patent Office

Office européen des brevets

(11) Numéro de publication : **0 089 270 B1**

(12) **FASCICULE DE BREVET EUROPÉEN**

(45) Date de publication du fascicule du brevet :
29.07.87

(21) Numéro de dépôt : 83400458.2

(22) Date de dépôt : 07.03.83

(51) Int. Cl.⁴ : **A 61 K 39/395**

(54) Médicaments cytotoxiques comportant au moins une immunotoxine et une amine.

(30) Priorité : 11.03.82 FR 8204119

(43) Date de publication de la demande :
21.09.83 Bulletin 83/38

(45) Mention de la délivrance du brevet :
29.07.87 Bulletin 87/31

(84) Etats contractants désignés :
AT BE CH DE FR GB IT LI LU NL SE

(56) Documents cités :
FR-A- 2 466 252
BIOLOGICAL ABSTRACTS, vol. 73, 1982, résumé no. 78102, Philadelphia, P.A., USA Y. J. SCHNEIDER et al.: "Effect of chloroquine and methylamine on endocytosis of fluoroscein-labeled control immunoglobulin G and of anti-plasma membrane immunoglobulin G by cultured fibroblasts"

(73) Titulaire : SANOFI, Société dite:
40, Avenue George V
F-75008 Paris (FR)

(72) Inventeur : Jansen, Franz
Chemin des Sesquets
F-34160 Assas (FR)
Inventeur : Gros, Pierre
18 rue des Muriers
F-34100 Montpellier (FR)

(74) Mandataire : Gillard, Marie-Louise et al
Cabinet Beau de Loménie 55, Rue d'Amsterdam
F-75008 Paris (FR)

## Description

L'invention concerne des médicaments cytotoxiques, caractérisés en ce qu'ils contiennent en association au moins une immunotoxine obtenue par couplage, par liaison covalente, de la chaîne A de la ricine avec des anticorps ou fragments d'anticorps dirigés contre un antigène porté par les cellules à détruire, et une amine de formule $R_1NHR_2$ dans laquelle $R_1$ désigne l'hydrogène ou un groupe alkyle ayant 1 à 4 atomes de carbone, $R_2$ désigne un groupe alkyle ayant 1 à 4 atomes de carbone ou l'ensemble $R_1NHR_2$ représente l'amino-1 adamantane, ladite amine étant utilisée sous forme d'amine ou d'un sel d'amine pharmaceutiquement acceptable, elle concerne également l'utilisation d'une amine de formule $R_1NHR_2$ dans laquelle $R_1$ désigne l'hydrogène ou un groupe alkyle ayant 1 à 4 atomes de carbone, $R_2$ désigne un groupe alkyle ayant 1 à 4 atomes de carbone ou l'ensemble $R_1NHR_2$ représente l'amino-1 adamantane, ou d'un de ses sels pharmaceutiquement acceptables en association avec au moins une immunotoxine obtenue par couplage, par liaison covalente, de la chaîne A de la ricine avec des anticorps ou fragments d'anticorps dirigés contre un antigène porté par les cellules à détruire, pour la préparation d'un médicament à effet cytotoxique potentialisé.

Dans les demandes de brevets français antérieures portant notamment les numéros FR-A-2 437 213, FR-A-2 466 252, FR-A-2 504 010 et FR-A-2 516 794, on a décrit la préparation de produits anticancéreux dits conjugués obtenus par couplage, par liaison covalente, de la chaîne A de la ricine avec des anticorps ou fragments d'anticorps dirigés contre un antigène porté par une cellule à détruire. Les produits de ce type sont désignés dans la présente demande sous le nom générique d'immunotoxines.

Dans la demande française FR-A-2 516 794 on a décrit en outre la propriété des ions ammonium (sous la forme d'un quelconque de leurs sels et en particulier le chlorure) de potentialiser de façon efficace l'action cytotoxique de ces immunotoxines.

Dans la demande de brevet français FR-A-2 521 010, la demanderesse a décrit la propriété des substances appartenant à la classe des ionophores carboxyliques de potentialiser l'activité des immunotoxines et d'accélérer leur cinétique d'action.

La présente invention a pour objet la préparation de médicaments cytotoxiques puissants utilisant la potentialisation des effets cytotoxiques sélectifs des immunotoxines décrites dans les demandes de brevets antérieures ; il a été trouvé en effet que les amines définies ci-dessus utilisées sous la forme d'un de leurs sels pharmaceutiquement acceptables et à des doses où elles ne présentent elles-mêmes aucune cytotoxicité propre pour les lignées étudiées étaient des potentialisateurs extrêmement puissants et des accélérateurs de l'effet cytotoxique des immunotoxines.

Les exemples suivants permettent de mieux comprendre la portée de l'invention.

### Exemple 1

Cet exemple démontre la potentialisation de la cytotoxicité sélective de l'immunotoxine anti-T65 (telle que décrite dans la demande FR-A-2 516 794 de la demanderesse) vis-à-vis des cellules lymphoblastoïdes T humaines de la lignée CEM portant l'antigène T65 par la méthylamine.

Dans ces expériences, la cytotoxicité a été évaluée par la mesure de l'incorporation de $^{14}$C-leucine par les cellules après 18 h d'incubation à 37 °C en présence de quantités connues de l'immunotoxine étudiée, ou de substances cytotoxiques de référence, en absence ou en présence de méthylamine.

1 — Potentialisation de l'effet cytotoxique par la méthylamine.

Les résultats de ces expériences sont présentés sous la forme de courbes dose/effet présentant en ordonnée l'effet cytotoxique évalué comme indiqué ci-dessus par l'incorporation du traceur, calculée en % de la valeur obtenue sur les cellules témoins sans substance cytotoxique et en abscisse les concentrations molaires en sous-unité toxique des substances cytotoxiques étudiées. La méthylamine a été testée à la concentration de 10 millimolaires. Il a été préalablement vérifié que la méthylamine n'est pas spontanément cytotoxique pour les cellules employées à la concentration indiquée.

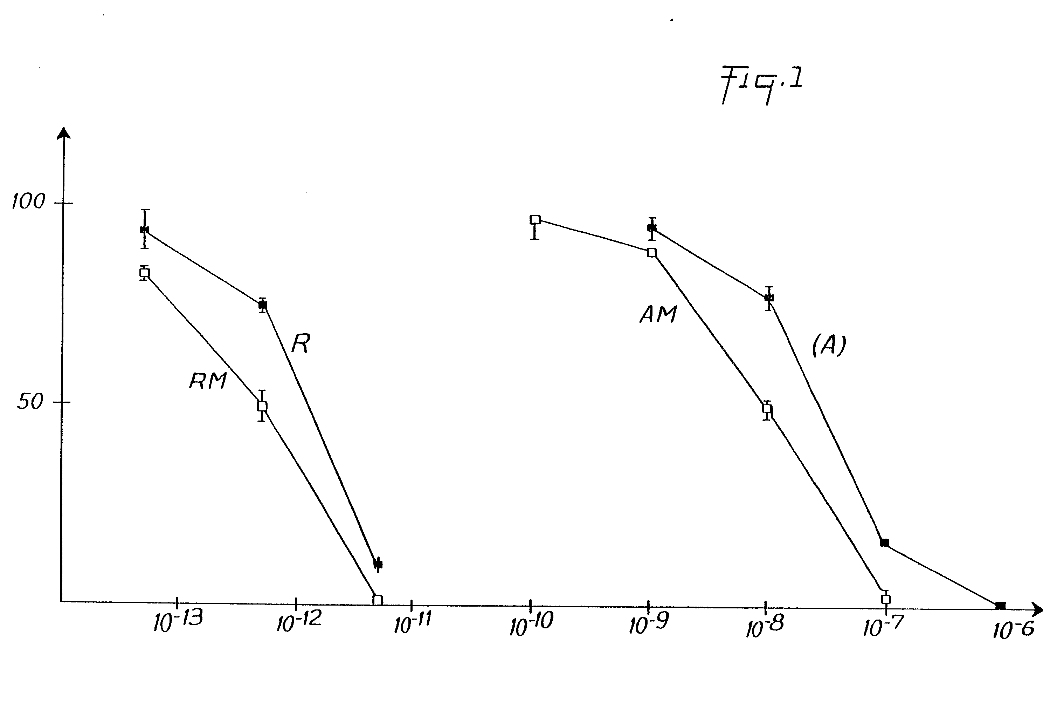

Sur la figure 1 on a représenté respectivement les résultats obtenus pour
la chaîne A de la ricine (A)
la ricine (R)
la chaîne A de la ricine et la méthylamine (AM)
la ricine et la méthylamine (RM).

La figure 1 montre l'effet de la méthylamine sur la cytotoxicité propre de la ricine et de la chaîne A isolée, prises comme substances de référence. Les valeurs des concentrations molaires (C150) correspondant à 50 % d'inhibition d'incorporation du traceur sont indiquées dans le tableau I.

# 0 089 270

Tableau I

| Substances testées | Avec méthylamine 10 mM | Sans méthylamine |
|---|---|---|
| Ricine | $5.10^{-13}$ M | $1,2.10^{-12}$ M |
| Chaîne A | $1.10^{-8}$ M | $2,6.10^{-8}$ M |

Ces résultats démontrent un très faible effet potentialisateur de la méthylamine sur la cytotoxicité de la ricine (facteur de potentialisation de 2,4) et de la chaîne A (facteur de potentialisation de 2,6).

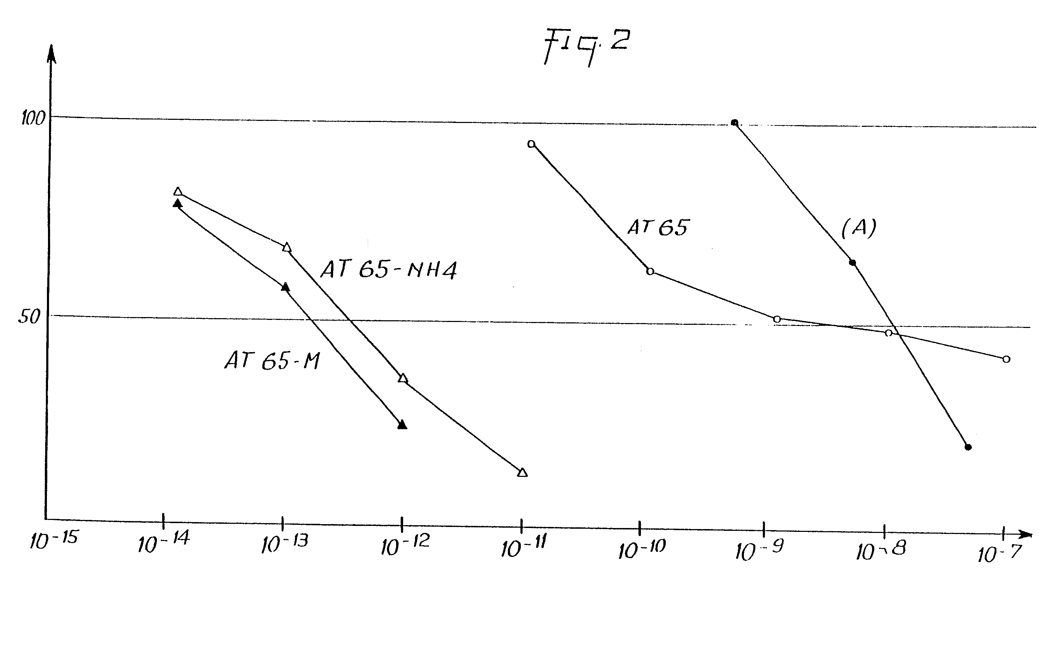

Sur la figure 2 on a représenté respectivement les résultats obtenus pour :

la chaîne A de la ricine (A)

le conjugué anti-T65 (AT65)

le conjugué anti-T65 en mélange avec l'ion ammonium (AT65-NH$_4$)

le conjugué anti-T65 en mélange avec la méthylamine (AT65-M)

La figure 2 montre l'effet potentialisateur comparé de l'ion $NH_4^+$ (10 mM) et de la méthylamine (10 mM) sur la cytotoxicité de l'immunotoxine anti-T65 vis-à-vis des cellules de la lignée CEM. Les valeurs des concentrations molaires (C150) correspondant à 50 % d'inhibition d'incorporation du traceur sont rappelées dans le tableau II.

Tableau II

| Substances potentialisatrices testées | CI50 |
|---|---|
| Aucune | $5.10^{-9}$ M |
| $NH_4^+$ 10 mM | $3.10^{-13}$ M |
| Méthylamine 10 mM | $1,5.10^{-13}$ M |

Ces résultats montrent que l'effet potentialisateur de la méthylamine est voisin de celui de l'ion ammonium et de l'ordre d'un facteur de 13 000. Ce facteur est considérablement plus élevé que ceux observés avec la ricine ou la chaîne A isolée. Ceci signifie qu'en présence de méthylamine l'immunotoxine anti-T65 est, vis-à-vis de sa cible spécifique, un agent cytotoxique près de 3 fois plus puissant que la ricine elle-même.

En outre, la méthylamine présente la propriété remarquable, non seulement de potentialiser l'activité, mais aussi d'augmenter la sélectivité de l'immunotoxine. Si l'on prend en effet comme critère de sélectivité d'action de l'immunotoxine le rapport des C150 de la chaîne A libre et de l'immunotoxine, ce rapport est de l'ordre de 5 en l'absence de méthylamine et voisin de 65 000 en présence de méthylamine.

2 — Accélération des cinétiques de cytotoxicité par la méthylamine.

L'effet de la méthylamine ne se limite pas à accroître d'une façon considérable l'activité cytotoxique des immunotoxines. Cette substance permet aussi d'accélérer de façon très importante la cinétique de cytotoxicité des immunotoxines, comme le montre l'expérience suivante.

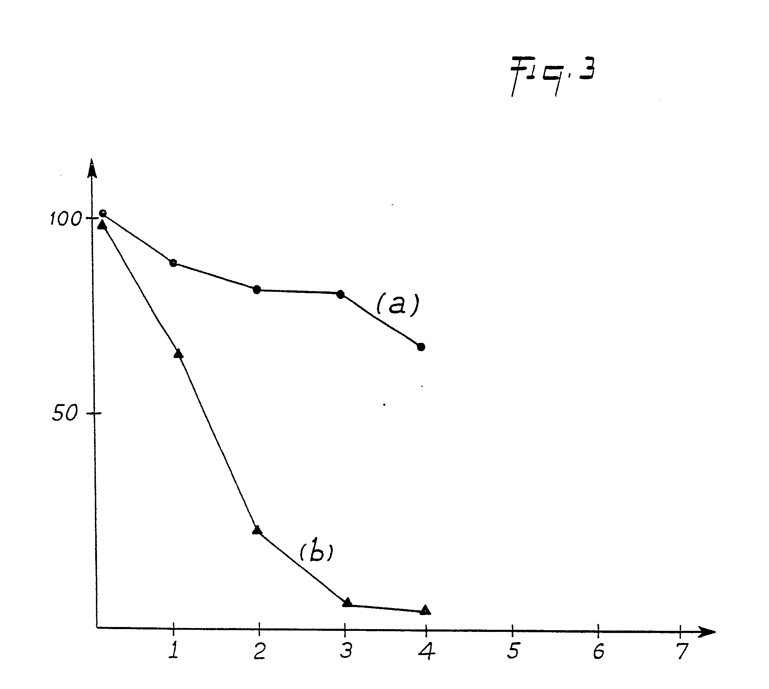

Dans cette expérience, on a mesuré comme précédemment l'incorporation de traceur radioactif dans les cellules mais cette fois en fonction du temps d'incubation des cellules avec l'immunotoxine, en absence et en présence de méthylamine 10 mM comme potentialisateur. Cette expérience a été réalisée sur le modèle cellulaire constitué par la lignée lymphoblastoïde humaine CEM avec l'immunotoxine anti-T65 à la concentration de 50 mM. Les résultats sont présentés dans la figure 3.

Sur cette figure on a représenté les résultats obtenus en portant en ordonnée le % d'incorporation de $^{14}$C-leucine (% des témoins) et en abscisse le temps (en heures).

Pour cette lignée, il apparaît qu'en l'absence de potentialisation la cinétique de cytotoxicité est très lente comme le montre la courbe a. (D'autres expériences dans les mêmes conditions ont montré que le temps nécessaire à obtenir 50 % de réduction de l'incorporation du traceur était de l'ordre de 20 h). Par contre, en présence de méthylamine une accélération spectaculaire de la cinétique se manifeste (courbe b) puisque le temps nécessaire à obtenir 50 % d'inhibition d'incorporation est alors de l'ordre de 1,5 h seulement.

Un tel effet d'accélération est de la plus haute importance pour toutes applications des immunotoxines et en particulier pour les applications thérapeutiques in vivo car la rapidité d'action du médicament est toujours un facteur très favorable à l'efficacité du traitement.

3

## Exemple 2

En opérant comme dans l'exemple 1 mais en faisant varier l'amine utilisée on a déterminé la potentialisation de l'effet cytotoxique par une amine (exemple 1 point 1). L'effet potentialisateur, exprimé en C150 (cf. tableau I) avec l'immunotoxine anti-T65, est représenté dans le tableau III.

Tableau III

| Potentialisateur | Concentration molaire amine | CI50 (molaire) |
|---|---|---|
| sans | - | $5.10^{-9}$ |
| diméthylamine | $10^{-2}$ | $3.10^{-12}$ |
| amino-1 adamantane | $10^{-3}$ | $8.10^{-12}$ |

On peut donc utiliser en tant que médicament en thérapeutique humaine l'association constituée par une immunotoxine et une amine telle que définie sous la forme de l'un quelconque de ses sels. Il peut être utilisé pour le traitement des affections, cancéreuses ou non, qui seraient sensibles à l'anticorps utilisé pour la préparation de l'immunotoxine.

Visant à éliminer la totalité des cellules cancéreuses le traitement devra être effectué avec une dose suffisante d'immunotoxine associée avec une quantité d'amine pouvant varier de 10 mg à 2 g (exprimés en base) à chaque administration d'immunotoxine. La durée du traitement devra être déterminée dans chaque cas en fonction du sujet et de la nature de l'affection à traiter.

Les médicaments selon l'invention sont conditionnés pour être utilisés par voie injectable et de préférence par voie intraveineuse.

De préférence les constituants de l'association seront conservés séparément et mélangés, seulement au moment de l'emploi, dans la seringue ou le solvant de perfusion.

**Revendications** (pour les Etats contractants : BE, CH, DE, FR, GB, IT, LI, LU, NL, SE)

1. Médicaments cytotoxiques, caractérisés en ce qu'ils contiennent en association au moins une immunotoxine obtenue par couplage, par liaison covalente, de la chaîne A de la ricine avec des anticorps ou fragments d'anticorps dirigés contre un antigène porté par les cellules à détruire, et une amine de formule $R_1NHR_2$ dans laquelle $R_1$ désigne l'hydrogène ou un groupe alkyle ayant 1 à 4 atomes de carbone, $R_2$ désigne un groupe alkyle ayant 1 à 4 atomes de carbone ou l'ensemble $R_1NHR_2$ représente l'amino-1 adamantane, ladite amine étant utilisée sous forme d'amine ou d'un sel d'amine pharmaceutiquement acceptable.

2. Médicaments selon la revendication 1, caractérisés en ce que l'amine utilisée est la méthylamine ou l'un de ses sels.

3. Médicaments selon la revendication 1, caractérisés en ce qu'ils sont conditionnés pour être administrés par voie injectable, de préférence par voie intraveineuse.

4. Utilisation d'une amine de formule $R_1NHR_2$ dans laquelle $R_1$ désigne l'hydrogène ou un groupe alkyle ayant 1 à 4 atomes de carbone, $R_2$ désigne un groupe alkyle ayant 1 à 4 atomes de carbone ou l'ensemble $R_1NHR_2$ représente l'amino-1 adamantane, ou d'un de ses sels pharmaceutiquement acceptables en association avec au moins une immunotoxine obtenue par couplage, par liaison covalente, de la chaîne A de la ricine avec des anticorps ou fragments d'anticorps dirigés contre un antigène porté par les cellules à détruire, pour la préparation d'un médicament à effet cytotoxique potentialisé.

**Revendication** (pour l'Etat contractant AT)

Utilisation d'une amine de formule $R_1NHR_2$ dans laquelle $R_1$ désigne l'hydrogène ou un groupe alkyle ayant 1 à 4 atomes de carbone, $R_2$ désigne un groupe alkyle ayant 1 à 4 atomes de carbone ou l'ensemble $R_1NHR_2$ représente l'amino-1 adamantane ou d'un de ses sels pharmaceutiquement acceptables en association avec au moins une immunotoxine obtenue par couplage, par liaison covalente, de la chaîne A de la ricine avec des anticorps ou fragments d'anticorps dirigés contre un antigène porté par les cellules à détruire, pour la préparation d'un médicament à effet cytotoxique potentialisé.

**Claims** (for the Contracting States : BE, CH, DE, FR, GB, IT, LI, NU, NL, SE)

1. Cytotoxic drugs, characterized in that they contain in association at least one immunotoxin obtained by coupling, by covalent bond, of the A chain of ricin with antibodies or fragments of antibodies directed against an antigen carried by the cells to be destroyed, and one amine of formula $R_1NHR_2$ in which $R_1$ designates hydrogen or an alkyl group having from 1 to 4 atoms of carbon, and $R_2$ designates an alkyl group having from 1 to 4 atoms of carbon, or the assembly $R_1NHR_2$ represents 1-amino adamantane, said amine being used in the form of amine or a pharmaceutically acceptable amine salt.

2. Drugs according to claim 1, characterized in that the amine used is methylamine or one of its salts.

3. Drugs according to claim 1, characterized in that they are packed to be administered by the injectable route, preferably by the intravenous route.

4. Use of an amine of formula $R_1NHR_2$ in which $R_1$ designates hydrogen or an alkyl group having from 1 to 4 atoms of carbon, $R_2$ designates an alkyl group having from 1 to 4 atoms of carbon or the assembly $R_1NHR_2$ represents 1-amino adamantane, or one of its pharmaceutically acceptable salts in association with at least one immunotoxin obtained by coupling, by covalent bond, of the A chain of ricin with antibodies or fragments of antibodies directed against an antigen carried by the cells to be destroyed, for the preapration of a drug having a potentialized cytotoxic effect.

**Claim** (for the Contracting State AT)

Use of an amine of formula $R_1NHR_2$ in which $R_1$ designates hydrogen or an alkyl group having from 1 to 4 atoms of carbon, $R_2$ designates an alkyl group having from 1 to 4 atoms of carbon or the assembly $R_1NHR_2$ represents 1-amino adamantane or one of its pharmaceutically acceptable salts in association with at least one immunotoxin obtained by coupling, by covalent bond, of the A chain of ricin with antibodies or fragments of antibodies directed against an antigen carried by the cells to be destroyed, for the preparation of a drug having a potentialized cytotoxic effect.

**Patentansprüche** (für die Vertragsstaaten : BE, CH, DE, FR, GB, IT, LI, LU, NL, SE)

1. Zytotoxische Medikamente, dadurch gekennzeichnet, daß sie in Kombination mindestens ein Antitoxin, das durch Kopplung mittels kovalenter Bindung der Kette A von Ricin mit Antikörpern oder Antikörperfragmenten, die gegen ein von den zu zerstörenden Zellen getragenes Antigen gerichtet sind, erhalten ist, und ein Amin der Formel $R_1NHR_2$, worin $R_1$ Wasserstoff oder eine Alkylgruppe mit 1 bis 4 Kohlenstoffatomen bezeichnet, $R_2$ eine Alkylgruppe mit 1 bis 4 Kohlenstoffatomen darstellt, oder $R_1HR_2$ als ganzes für 1-Amino-adamantan steht, enthalten, wobei das Amin in Form des Amins oder eines pharmazeutisch akzeptablen Aminsalzes verwendet ist.

2. Medikamente nach Anspruch 1, dadurch gekennzeichnet, daß das verwendete Amin Methylamin oder eines seiner Salze ist.

3. Medikamente nach Anspruch 1, dadurch gekennzeichnet, daß sie zur Verabreichung auf Injektionsweg, vorzugsweise intravenösem Weg, konditioniert sind.

4. Verwendung eines Amins der Formel $R_1NHR_2$, worin $R_1$ Wasserstoff oder eine Alkylgruppe mit 1 bis 4 Kohlenstoffatomen bezeichnet, $R_2$ eine Alkylgruppe mit 1 bis 4 Kohlenstoffatomen darstellt, oder $R_1NHR_2$ als ganzes für 1-Amino-adamantan steht, oder eines seiner pharmazeutisch akzeptablen Salze, in Kombination mit mindestens einem Antitoxin, das durch Kopplung mittels kovalenter Bindung der Kette A von Ricin mit Antikörpern oder Antikörperfragmenten, die gegen ein von den zu zerstörenden Zellen getragenes Antigen gerichtet sind, erhalten ist, zur Herstellung eines Medikaments mit potenzierter zytotoxischer Wirkung.

**Patentanspruch** (für den Vertragsstaat AT)

Verwendung eines Amins der Formel $R_1NHR_2$, worin $R_1$ Wasserstoff oder eine Alkylgruppe mit 1 bis 4 Kohlenstoffatomen bezeichnet, $R_2$ eine Alkylgruppe mit 1 bis 4 Kohlenstoffatomen darstellt, oder $R_1NHR_2$ als ganzes für 1-Amino-adamantan steht, oder eines seiner pharmazeutisch akzeptablen Salze, in Kombination mit mindestens einem Antitoxin, das durch Kopplung mittels kovalenter Bindung der Kette A von Ricin mit Antikörpern oder Antikörperfragmenten, die gegen ein von den zu zerstörenden Zellen getragenes Antigen gerichtet sind, erhalten ist, zur Herstellung eines Medikaments mit potenzierter zytotoxischer Wirkung.

Fig.1

Fig. 2

AT 65-NH4

AT 65-M

AT 65

(A)

100

50

2

0 089 270

10⁻¹⁵  10⁻¹⁴  10⁻¹³  10⁻¹²  10⁻¹¹  10⁻¹⁰  10⁻⁹  10⁻⁸  10⁻⁷

Fig. 3